# EUROPEAN PATENT SPECIFICATION

(11) **EP 3 155 964 B1**
(45) Date of publication and mention of the grant of the patent: **06.07.2022**
(21) Application number: 16188684.1
(22) Date of filing: 14.09.2016
(51) Int. Cl.: A61B 5/364

(54) **A METHOD AND A MONITORING DEVICE FOR ANALYZING ECG DATA BY COMPARING CONSECUTIVE TIME INTERVALS**
EIN VERFAHREN UND EIN ÜBERWACHUNGSGEÄT ZUR ANALYSE VON EKG-DATEN DURCH VERGLEICHEN VON AUFEINANDERFOLGENDEN ZEITINTERVALLEN
UN PROCÉDÉ ET UN DISPOSITIF DE SURVEILLANCE POUR L'ANALYSE DE DONNÉES ECG PAR LA COMPARAISON D'INTERVALLES DE TEMPS CONSÉCUTIFS

(30) Priority: 30.09.2015 US 201562234700 P
(43) Date of publication of application: 19.04.2017
(73) Proprietor: BIOTRONIK SE & Co. KG, 12359 Berlin (DE)
(72) Inventor: Bellock, Steven, Wilsonville, OR Oregon 97070 (US); Whittington, R. Hollis, Portland, OR Oregon 97202 (US); Garner, Garth, Tigard, OR Oregon 97224 (US)
(74) Representative: Biotronik Corporate Services SE

(56) References cited:
- US-A1- 2002 177 784
- US-A1- 2010 317 979
- US-A1- 2012 238 892
- US-B1- 6 496 722

## Description

The invention generally relates to implantable cardiac devices, including monitoring devices, pacemakers, defibrillators and cardioverters, which monitor, detect and classify cardiac events, for example atrial tachyarrhythmias. More particularly, embodiments of the invention relate to a method and device for detecting atrial fibrillation by evaluating ventricular signals.

Various types of heart rhythm disorders are known, some of which are life-threatening and require immediate attention and treatment, such as ventricular fibrillation. Other arrhythmias may require treatment, and/or may be symptomatic of other underlying conditions requiring treatment, but are typically not immediately life-threatening. Atrial fibrillation (AF), for example, is a relatively common cardiac arrhythmia associated with increased risk of stroke and death. Other less-common cardiac arrhythmias that would be beneficial to diagnose include, but are not limited to, paroxysmal ventricular tachycardia, paroxysmal atria tachycardia, supraventricular tachycardia, and sinus tachycardia. Although the following disclosure will, for simplicity, refer to AF, it will be understood that the disclosed methods are also generally applicable to other cardiac arrhythmias.

AF can be either symptomatic or asymptomatic, and can be paroxysmal or persistent. AF is usually diagnosed when a patient exhibits associated symptoms or complications, such as palpations, congestive heart failure or stroke. AF may also be diagnosed incidentally during a routine medical evaluation. Patients with asymptomatic paroxysmal AF may be exposed to the risk of devastating consequences such as stroke, congestive heart failure, or tachycardia-mediated cardiomyopathy, for years before a definitive diagnosis of AF can be made.

In the field of automated detection of dangerous arrhythmias, there are previously proposed methods for detecting atrial tachyarrhythmias.

It is known from the prior art to use a so called "X-out-of-Y" criterion to detect an ongoing atrial tachyarrhythmia. The U.S. Pat. No. 6,671,548 B1 for example describes use of such a "X-out-of-Y" criterion. This criterion declares detection of an atrial tachyarrhythmia when X number of intervals among most recent Y number of atrial intervals are found to be shorter than an interval limit corresponding to the tachyarrhythmia rate limit. The numbers X, Y and the tachyarrhythmia rate limit may be user defined, e.g., pre-defined or may be programmable. As is clear, the "X-out-of-Y" criterion accommodates for undersensing of some of the atrial events.

US 6,496,722 B1 describes a method and a device for evaluating electrocardiograms in the area of extrasystoles, whereby the time intervals between successive heartbeats directly before and after an extrasystole are determined.

A problem in the detection of dangerous arrhythmias from heart activity data such as electrocardiogram (ECG) data is that a generally healthy heart may often exhibit some variability in the ECG data that can confuse or mislead automated detection algorithms. Relatively benign variability may include premature atrial contraction, premature ventricular contraction, and normal sinus arrhythmia. Common types of arrhythmia due to premature ventricular contractions are for instance continuous alternations of long and short heartbeats with an inherent regularity, which are called bigeminy, trigeminy, and so forth according to the number of ventricular contractions. A major problem with screening for potentially dangerous heart rhythm irregularities, such as atrial fibrillation, ventricular tachycardia, and the like, is that existing detection methods lack sufficient specificity, meaning that existing detection methods are not able to differentiate dangerous cardiac arrhythmias from benign arrhythmias to a sufficient extent. Such existing detection methods produce a high rate of false positives, generating incorrect diagnosis, anxiety in healthy subjects, causing expensive technician review, and possibly spurring unnecessary, expensive, potentially uncomfortable, and inconvenient additional testing.

The disclosure is in general directed to correctly detect and differentiate between life-threatening cardiac arrhythmias such as AF from arrhythmias due to periodic premature events, such as bigeminy, trigeminy, and other periodic rhythms.

According to the invention, a method is disclosed for analyzing ECG data. The method comprises the steps of:
- detecting points in the ECG data which represent ventricular activity,
- measuring time intervals between each two consecutive points in the ECG data which represent ventricular activity,
- evaluating the time intervals within a set of time intervals by
- computing at least one comparative dimension for at least one time interval subset, wherein the time interval subset comprises at least two time intervals, the comparative dimension represents a difference of the interval length between the time intervals of the time interval subset, and wherein a fixed number N of time intervals lies between each two time intervals of the time interval subset and wherein N ≥ 1.

The said set of time intervals involved in the presented method comprises at least three time intervals. Also, the points in the ECG data which represent ventricular activity may be QRS complexes, or in particular, R-waves.

In one embodiment, the method according to the invention further includes the step of delivering stimulation to a heart by a heart monitoring device, the stimulation being at least partially dependent on the indication of pathology.

In a preferred embodiment of the disclosed method for analyzing ECG data, an additional comparative dimension for at least one time interval subset is computed for which N = 0 within the set of time intervals.

Preferably, the said comparative dimension involved in the disclosed method is computed by generation of at least a difference, a sum, a ratio, a product, a mean value, a deviation, or a variance.

According to embodiments of the inventive method, all computed comparative dimensions with equal N are considered as a group within the set of time intervals, and for each group, a number is calculated for those comparative dimensions which fulfil an instability criterion.

Furthermore, according to one embodiment of the disclosed method, each calculated number is compared with at least one threshold respectively and the set of time intervals is regarded as irregular, when at least one calculated number exceeds at least one threshold respectively or when all calculated numbers exceed at least one threshold respectively.

According to a preferred embodiment of the presented method, a quality is assigned to at least a part of the ECG data, when a count of consecutive sets of time intervals which are regarded as irregular exceeds a value A and/or when within a totality X of sets of time intervals, the count of consecutive sets of time intervals which are regarded as irregular exceeds the value A.

According to one embodiment of the disclosed method, the assignment of a quality to at least a part of the ECG data is terminated, when a count of consecutive sets of time intervals which are regarded as not irregular exceeds a value B and/or when within a totality Y of sets of time intervals, the count of consecutive sets of time intervals which are regarded as not irregular exceeds the value B.

For instance, the assignment of a quality according to the disclosed method can comprise marking the respective ECG data as being indicative of a cardiac arrhythmia which can typically be identified on the ECG by irregular ventricular activity, as for example AF or atrial flutter.

In a preferred embodiment of the presented method, the instability criterion is fulfilled when the comparative dimension exceeds a limit. The limit can also be calculated dynamically according to the computed comparative dimensions.

Preferably, the inventive method is employed in a device for analyzing ECG data during the detection phase for a cardiac arrhythmia as AF. The method can also be applied in the initial phase for confirmation of a cardiac arrhythmia within such devices.

Another objective of the invention is to provide a device for monitoring the heart activity of a living being, for example an implantable cardiac device, such as a monitoring device, especially a monitoring device without atrial electrodes, but also such as a pacemaker, a defibrillator or a cardioverter, for evaluating cardiac events, such as ventricular signals, for detecting atrial arrhythmia such as AF or atrial flutter, wherein the device comprises
- at least two electrodes for recording a signal which represents the heart activity,
- a power supply,
- a memory unit for storing the signal, and
- a signal evaluation unit, which is configured for detecting ventricular activity in the signal, measuring time intervals between each two consecutive ventricular activities, evaluating the time intervals within a set of time intervals. The evaluation of time intervals within a set of time intervals is performed by
   - computing at least one comparative dimension for at least one time interval subset, wherein
   - the time interval subset comprises at least two time intervals,
   - the comparative dimension represents a difference of the interval length between the time intervals of the time interval subset, and wherein
   - a fix number M of time intervals lies between each two time intervals of the time interval subset and wherein M ≥ 1.

The said set of time intervals according to the disclosed device comprises at least three time intervals. Also, the ventricular activity in the signal can be represented by QRS-complexes, or in particular, by R-waves.

According to an embodiment of the disclosed device, the signal evaluation unit is further configured to compute an additional comparative dimension for which M =0 for at least one time interval subset within the set of time intervals.

Preferably, the comparative dimension according to the presented device is computed by generation of at least a difference, a sum, a ratio, a product, a mean value, a deviation, or a variance.

Preferably, the signal evaluation unit is moreover configured to consider all computed comparative dimensions with equal N within the set of time intervals as a group, and calculate a number for each group for those comparative dimensions which fulfil at least one instability criterion.

In one embodiment of the disclosed device, the signal evaluation unit is further configured to regard the set of time intervals as irregular, when at least one calculated number exceeds at least one threshold or when all calculated numbers exceed at least one threshold.

According to one embodiment of the present invention, the evaluation unit is configured to recognize a pathological state of the heart activity, when a count of consecutive sets of time intervals which are regarded as irregular exceeds a value C and/or when within a totality Z of sets of time intervals, the count of consecutive sets of time intervals which are regarded as irregular exceeds the value C.

Preferably, the signal evaluation unit is further configured to terminate recognition of a pathological state of the heart activity, when a count of consecutive sets of time intervals which are regarded as not irregular exceeds a value D and/or when within a totality E of sets of time intervals, the count of consecutive sets of time intervals which are regarded as not irregular exceeds the value D.

For instance, the recognition of a pathological state of the heart activity according to the disclosed device can comprise marking the respective ECG data as being indicative of a cardiac arrhythmia which can typically be identified on the ECG by irregular ventricular activity, as for example AF or atrial flutter.

In a preferred embodiment of the presented device, the instability criterion is fulfilled when the comparative dimension exceeds a limit. The limit can also be calculated dynamically according to the computed comparative dimensions.

In another embodiment of the presented device, the device comprises a telemetry module for wireless communication e.g. via radio frequency (RF) fields, changing electric and/or magnetic fields. For example, the disclosed device may perform wireless data transmission with a remote monitoring system, including an external device which may process the data from the cardiac implant and transmit the data to a further remote system, as for instance a clinical data center. In the remote system, the data from the cardiac device may be stored, post-processed, analyzed and monitored over a longer period of time.

Preferably, there may be a time period of the detection during which rhythms due to premature ventricular contractions are considered and after which regularity is not used to exclude an arrhythmia episode such as AF. As a specific example, the detection is used in the first 5 minutes of AF detection in order to avoid that an AF episode is triggered by arrhythmias due to premature ventricular contractions in the beginning. Once an AF episode exceeds 5 minutes in duration, the detection may be disabled to avoid that a long true AF episode is terminated or discarded due to the presence of premature ventricular contractions in the middle of that episode.

Moreover, the presented invention can be applied for filtering out periodic rhythms such as rhythms due to premature ventricular contractions from ECG data. The corresponding ECG data may be stored and/or transferred to external systems to be reviewed by physicians.

The above and other aspects, features and advantages of at least one embodiment of the invention will be more apparent from the following more particular description thereof, presented in conjunction with the following drawings wherein:
- Fig. 1: depicts schematically a signal obtained via ECG data from a heart with regular heart rate;
- Fig. 2: depicts schematically an example ECG from a heart showing bigeminus beats;
- Fig. 3: depicts schematically an example ECG from a heart showing trigeminus beats;
- Fig. 4: depicts schematically an example ECG from a heart showing quadrigeminus beats;
- Fig. 5: depicts schematically an example ECG from a heart showing irregularities such as for the indication of AF;
- Fig. 6: shows a heart stimulator and a remote monitoring system according to one or more embodiments of the invention;
- Fig. 7: illustrates a heart stimulator connected to electrode leads that are placed in a heart according to one or more embodiments of the invention;
- Fig. 8: depicts a schematic block diagram of some components of the heart stimulator of figure 2 according to one or more embodiments of the invention.

The following description is of the best mode presently contemplated for carrying out at least one embodiment of the invention. This description is not to be taken in a limiting sense, but is made merely for the purpose of describing the general principles of the invention. The scope of the invention should be determined with reference to the claims.

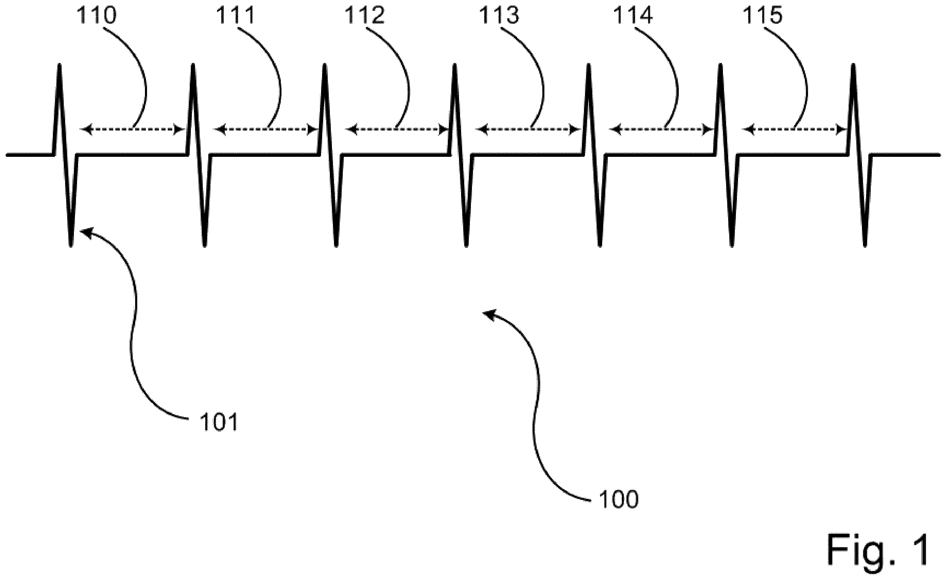

Figures 1 to 5 show schematic examples of different ECG signals 100, 200, 300, 400 and 500 with portions of the signal 101 representing ventricular activity. For those signals, exemplary applications of at least one embodiment of the invention are described.

According to at least one embodiment of the invention, an ECG signal is either measured by a heart activity monitoring device or generated of recorded ECG data. In Fig. 1, an exemplary ECG signal 100 of a heart with a regular heart rate is depicted schematically. In one or more embodiments of the invention, portions of the signal 101 representing ventricular activity are detected in the ECG signal, as for instance QRS complexes or R-waves. Those portions of the signal 101 can for example be determined via an amplitude threshold measurement, an analysis of the signal slope, or other appropriate techniques in signal processing and analysis. Moreover, time intervals 110....115 are measured between each of two portions of the signal 101.

According to the invention, the time intervals within a set of time intervals are evaluated, wherein the set of time intervals ('set' hereafter) comprises at least three time intervals. The evaluation is performed via computing a comparative dimension ('CD' hereafter) which represents a difference of the interval length between the time intervals of a time interval subset ('subset' hereafter). A subset comprises at least two time intervals. Furthermore, a fix number of N time intervals lies between each of two time intervals of the subset, wherein N ≥ 1. Furthermore, according to at least one embodiment of the present invention, for a set, an additional CD can be computed for at least one subset for which N = 0. CD can be computed by generating at least a difference, a sum, a ratio, a product, a mean value, a deviation, or a variance. It is included within the scope of the invention that CD may also be obtained using other types of mathematical methods and/or other operations from statistics.

At least one embodiment of the invention comprises that within the set of time intervals, all computed CD with equal N are considered as a group, and for each group, a number ('CNum_{N}' hereafter) is calculated for those CD which fulfil at least one instability criterion, for example when the CD exceeds a limit value. It is incorporated within the scope of the invention to apply different instability criterions for CD according to the boundary conditions of a specific implementation. It can be practicable to choose the instability criterion according to the type of CD and the required specificity and/or sensitivity for the arrhythmia detection. If CD is chosen for example as the difference value between two time intervals, a high difference value would imply that a change in the rate of ventricular activity has occurred. Therefore, it can be appropriate in that case to choose an instability criterion by means of a threshold value. If CD is chosen for example as mean value of at least two intervals, it can be appropriate to use an instability criterion by means of a reference value, wherein the reference value may be generated dynamically, for example out of previously computed mean values.

According to at least one embodiment of the invention, the set is regarded as irregular, when at least one CNum_{N} exceeds at least one threshold or when all CNum_{N} exceed at least one threshold. CNum_{N} can be taken for evaluating the ECG data in regard to arrhythmias as for example AF. As a practicle example, at least a part of the ECG data may be assigned as AF when a count of consecutive sets which are irregular due to their CNum_{N} exceeds a value A. Alternatively, at least a part of the ECG data may be assigned as AF when within a totality X of sets, the count of consecutive sets of time intervals which are regarded as irregular due to their CNum_{N} exceeds the value A. Vice versa, assigning the ECG data as AF can be terminated, when a count of consecutive sets intervals which are regarded as not irregular exceeds a value B and/or when within a totality Y of sets, the count of consecutive sets which are regarded as not irregular exceeds the value B. An appropriate threshold for CNum_{N} can be determined according to the required specificity and/or sensitivity of the arrhythmia detection.

The total number of computable CDs for one set depends on:
1. The total number of time intervals within a set;
2. The value(s) chosen for N;
3. The number of time intervals within a subset.

In the following, exemplary applications of the invention are illustrated for the different ECG signals in the Figs. 1-5. The examples relate to N = 0...3 and two time intervals within a subset. Of course, other numbers for N and/or the number of time intervals within a subset may be chosen in order to apply the presented invention.

In the examples, CD is computed as the difference of two time interval lengths. For the exemplary applications, the instability criterion for CD is assumed as fulfilled when CD is not zero. This instability criterion is chosen due to the sake of simplicity and clarity. Evidently, the choice of a suitable instability criterion depends on the applied type of CD and other parameters of the ECG signal analysis. In the examples, CNum_{N} represents the number of CD which are not zero for the group of CDs with the same N in a set.

For all examples,
- CDs are given as CD_{N,i} according to the corresponding N and subset number i within a sequence of subsets.
- Subsets are given as S_{N,i} according to the corresponding N and subset number i.
- CNums are given as CNum_{N} according to the corresponding N.

The ECG signal 100 in Fig. 1 shows an ECG signal with six time intervals 110...115 having the same length. If it is assumed that the shown six time intervals form a set, the following parameters are computed according to at least one embodiment of the invention:
With N = 0, five different subsets S_{0,i} can be formed consisting of:
   S_{0,1} : 110 and 111;
   S_{0,2} : 111 and 112;
   S_{0,3} : 112 and 113;
   S_{0,4} : 113 and 114.
   S_{0,5} : 114 and 115;
   All CD_{0,i} = 0; CNum₀ = 0.
With N = 1, four different subsets S_{1,i} can be formed consisting of:
   S_{1,1} : 110 and 112;
   S_{1,2} : 111 and 113;
   S_{1,3} : 112 and 114;
   S_{1,4} : 113 and 115.
   All CD_{1,i} = 0; CNum₁ = 0.
With N = 2, three different subsets S_{2,i} can be formed consisting of:
   S_{2,1} : 110 and 113;
   S_{2,2} : 111 and 114;
   S_{2.3} : 112 and 115.
   All CD_{2,i} = 0; CNum₂ = 0.
With N = 3, two different subsets S_{3,i} can be formed consisting of:
   S_{3,1} : 110 and 114;
   S_{3,2} : 111 and 115.
   All CD_{3,i} = 0; CNum₃ = 0.

Since all CD_{N,i} and CNum_{N} for the ECG signal in Fig. 1 would be zero, meaning that CD would not fulfil the instability criterion. Therefore, the assumed set shown in Fig. 1 is also not regarded as irregular according to at least one embodiment of the invention.

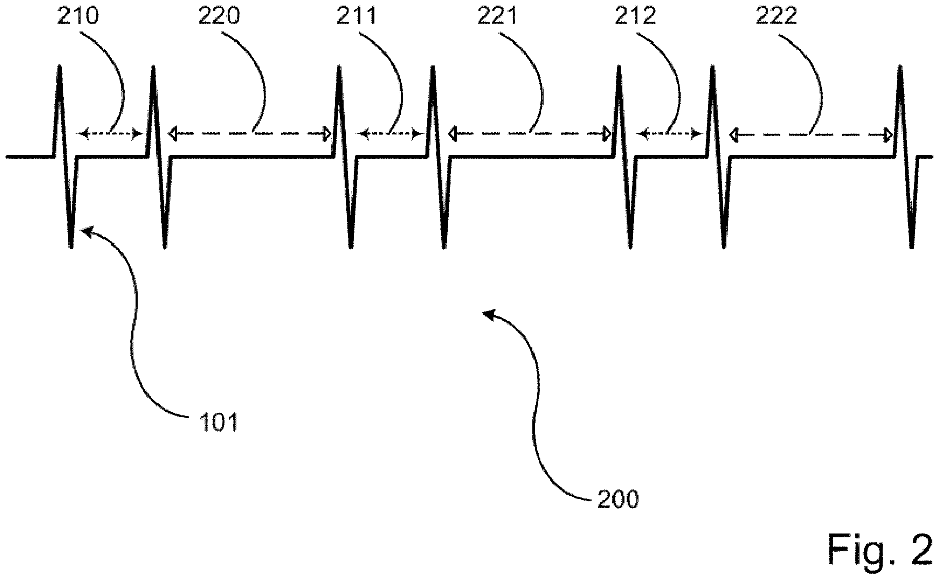

Fig. 2 depicts an exemplary ECG signal 200 with six time intervals, wherein 210, 211, 212 and 220, 221, 222 have the same length. The signal represents a schematic example ECG which shows the symptoms of bigeminy arrhythmia. Table 1 provides all S_{N,i} CD_{N,i} and and CNum_{N} (for N = 0...3 and the assumption the every S_{N,i} comprises two time intervals) which are obtained according to at least one embodiment according to the invention for the ECG signal in Fig. 2, in accordance the procedure presented for the ECG signal in Fig. 1.

**Table 1**

| **N** | **S_{N,i} (reference signs)** | **CD_{N,i} (0 or not 0)** | **CNum_{N}** |
|---|---|---|---|
| 0 | S_{0,1} : 210 and 220 | CD_{0,1} = not 0 | CNum₀ = 5 |
| | S_{0,2}: 220 and 211 | CD_{0,2} = not 0 | |
| | S_{0,3}: 211 and 221 | CD_{0,3} = not 0 | |
| | S_{0,4}: 221 and 212 | CD_{0,4} = not 0 | |
| | S_{0,5}: 212 and 222 | CD_{0,5} = not 0 | |
| 1 | S_{1,1} : 210 and 211 | CD_{1,1} = 0 | CNum₁ = 0 |
| | S_{1,2}: 220 and 221 | CD_{1,2}=0 | |
| | S_{1,3}: 211 and 212 | CD_{1,3} = 0 | |
| | S_{1,4}: 221 and 222 | CD_{1,4}=0 | |
| 2 | S_{2,1} : 210 and 221 | CD_{2,1} = not 0 | CNum₂ = 3 |
| | S_{2,2}: 220 and 212 | CD_{2,2} = not 0 | |
| | S_{2,3} : 211 and 222 | CD_{2,3} = not 0 | |
| 3 | S_{3,1} : 210 and 212 | CD_{3,1} = 0 | CNum₂ = 0 |
| | S_{3,2} : 220 and 222 | CD_{3,2} = 0 | |

| | | | |
|---|---|---|---|
| CNum_{N} = 0 is an indication for the example that a regularity is detected. In the case for Fig. 2, CNum_{N} = 0 has been computed for N = 1 and N = 3 using the invention according to at least one embodiment, meaning that a bigeminy structure has been detected and that the set may be regarded as not irregular. | | | |

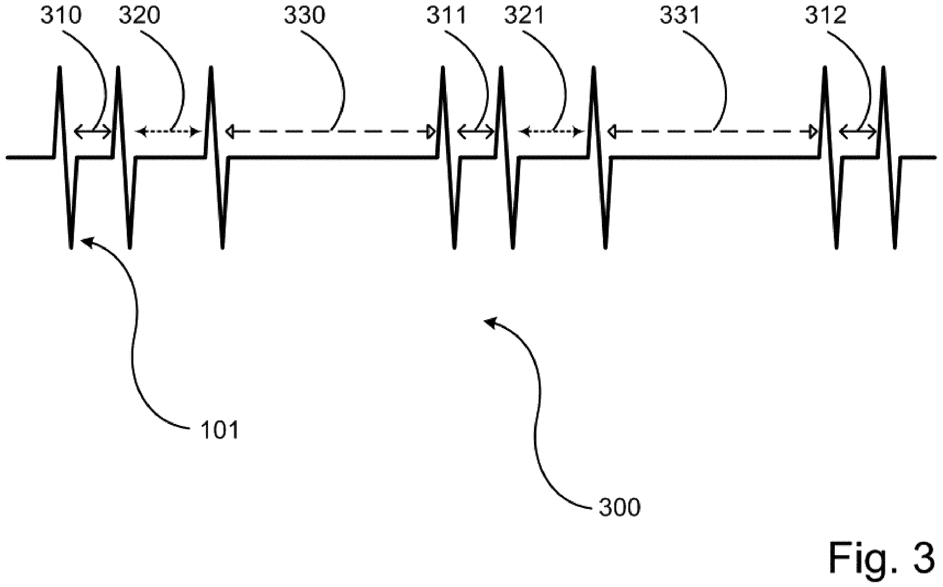

Fig. 3 depicts an exemplary ECG signal 300 with seven time intervals, wherein 310, 311, 312 and 320, 321 and 330, 331 have the same length. The signal represents a schematic example ECG which shows the symptoms of trigeminy arrhythmia. Table 2 provides all S_{N,i} CD_{N,i} and and CNum_{N} (for N = 0...3 and the assumption the every S_{N,i} comprises two time intervals) which are obtained according to at least one embodiment according to the invention for the ECG signal in Fig. 3, in accordance the procedure presented for the ECG signal in Fig. 1 and Fig. 2.

**Table 2**

| **N** | **S_{N,i} (reference signs)** | **CD_{N,i} (0 or not 0)** | **CNum_{N}** |
|---|---|---|---|
| 0 | S_{0,1} : 310 and 320 | CD_{0,1} = not 0 | CNum₀ = 6 |
| | S_{0,2}: 320 and 330 | CD_{0,2} = not 0 | |
| | S_{0,3} : 330 and 311 | CD_{0,3} = not 0 | |
| | S_{0,4}: 311 and 321 | CD_{0,4} = not 0 | |
| | S_{0,5}: 321 and 331 | CD_{0,5} = not 0 | |
| | S_{0,6}: 331 and 312 | CD_{0,6} = not 0 | |
| 1 | S_{1,1} : 310 and 330 | CD_{1,1} = not 0 | CNum₁ = 5 |
| | S_{1,2}: 320 and 311 | CD_{1,2} = not 0 | |
| | S_{1,3}: 330 and 321 | CD_{1,3} = not 0 | |
| | S_{1,4}: 311 and 331 | CD_{1,4} = not 0 | |
| | S_{1,5}: 321 and 312 | CD_{1,5} = not 0 | |
| 2 | S_{2,1} : 310 and 311 | CD_{2,1} = 0 | CNum₂ = 0 |
| | S_{2,2} : 320 and 321 | CD_{2,2} = 0 | |
| | S_{2,3}: 330 and 331 | CD_{2,3} = 0 | |
| | S_{2,4}: 311 and 312 | CD_{2,4} = 0 | |
| 3 | S_{3,1}: 310 and 321 | CD_{3,1} = not 0 | CNum₂ = 3 |
| | S_{3,2} : 320 and 331 | CD_{3,2} = not 0 | |
| | S_{3,3}: 330 and 312 | CD_{3,3} = not 0 | |

| | | | |
|---|---|---|---|
| CNum_{N} = 0 is an indication for the example that a regularity is detected. In the case for Fig. 3, CNum_{N} = 0 has been computed for N = 2 using the invention according to at least one embodiment, meaning that a trigeminus structure has been detected and that the set may be regarded as not irregular. | | | |

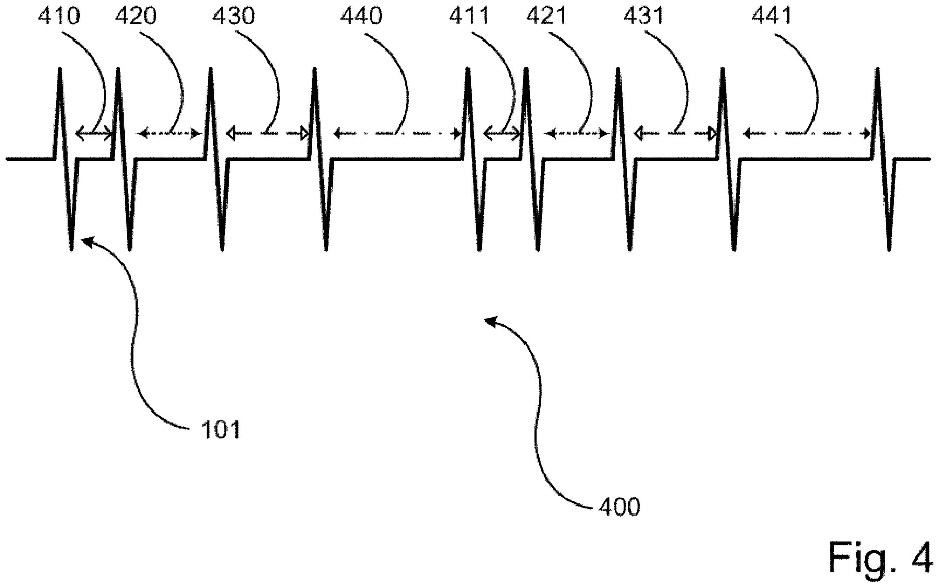

Fig. 4 depicts an exemplary ECG signal 400 with eight time intervals, wherein 410, 411 and 420, 421 and 430, 431 and 440, 441 have the same length. The signal represents a schematic example ECG which shows the symptoms of quadrigeminus arrhythmia. Table 3 provides all S_{N,i} CD_{N,i} and and CNum_{N} (for N = 0...3 and the assumption the every S_{N,i} comprises two time intervals) which are obtained according to at least one embodiment according to the invention for the ECG signal in Fig. 4, in accordance the procedure presented for the ECG signal in Fig. 1, Fig. 2 and Fig. 3.

**Table 3**

| **N** | **S_{N,i} (reference signs)** | **CD_{N,i} (0 or not 0)** | **CNum_{N}** |
|---|---|---|---|
| 0 | S_{0,1} : 410 and 420 | CD_{0,1} = not 0 | CNum₀ = 7 |
| | S_{0,2}: 420 and 430 | CD_{0,2} = not 0 | |
| | S_{0,3}: 430 and 440 | CD_{0,3} = not 0 | |
| | S_{0,4} : 440 and 411 | CD_{0,4} = not 0 | |
| | S_{0,5}: 411 and 421 | CD_{0,5} = not 0 | |
| | S_{0,6}: 421 and 431 | CD_{0,6} = not 0 | |
| | S_{0,7}: 431 and 441 | CD_{0,7} = not 0 | |
| 1 | S_{1,1} : 410 and 430 | CD_{1,1} = not 0 | CNum₁ = 6 |
| | S_{1,2} : 420 and 440 | CD_{1,2} = not 0 | |
| | S_{1,3} : 430 and 411 | CD_{1,3} = not 0 | |
| | S_{1,4} :440 and 421 | CD_{1,4} = not 0 | |
| | S_{1,5} :411 and 431 | CD_{1,5} = not 0 | |
| | S_{1,6} : 421 and 441 | CD_{1,6} = not 0 | |
| 2 | S_{2,1} : 410 and 440 | CD_{2,1} = not 0 | CNum₂ = 5 |
| | S_{2,2} : 420 and 411 | CD_{2,2} = not 0 | |
| | S_{2,3}: 430 and 421 | CD_{2,3} = not 0 | |
| | S_{2,4} : 440 and 431 | CD_{2,4} = not 0 | |
| | S_{2,5} : 411 and 441 | CD_{2,5} = not 0 | |
| 3 | S_{3,1} : 410 and 411 | CD_{3,1} = 0 | CNum₂ = 0 |
| | S_{3,2}: 420 and 421 | CD_{3,2} = 0 | |
| | S_{3,3}: 430 and 431 | CD_{3,3} = 0 | |
| | S_{3,4} : 440 and 441 | CD_{3,4} = 0 | |

| | | | |
|---|---|---|---|
| CNum_{N} = 0 is an indication for the example that a regularity is detected. In the case for Fig. 4, CNum_{N} = 0 has been computed for N = 3 using the invention according to at least one embodiment, meaning that a quadrigeminus structure has been detected and that the set may be regarded as not irregular. | | | |

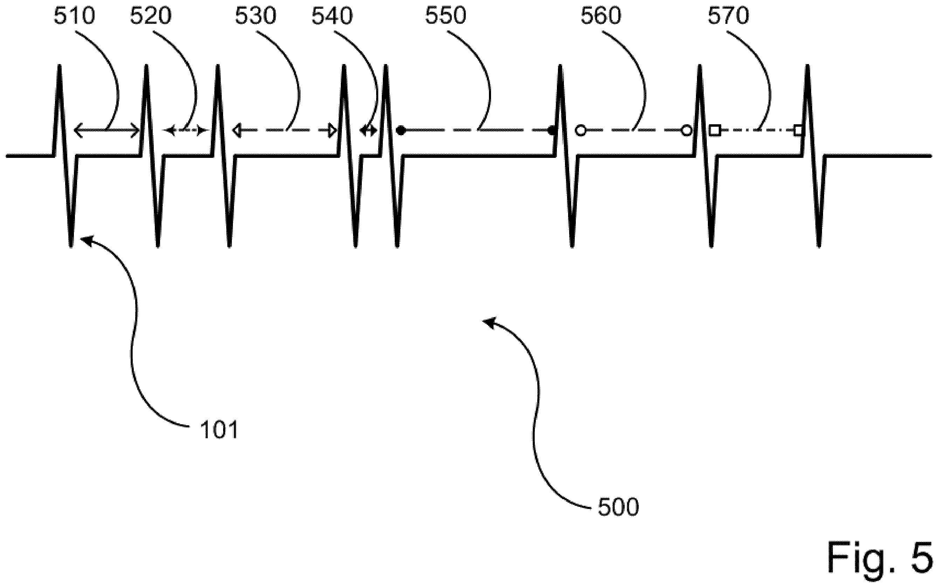

Fig. 5 depicts an exemplary ECG signal 500 with seven time intervals, wherein all time intervals have different lengths. The signal represents a schematic example ECG which shows the symptoms of an arrhythmia as AF. Table 4 provides all S_{N,i} CD_{N,i} and and CNum_{N} (for N = 0...3 and the assumption the every S_{N,i} comprises two time intervals) which are obtained according to at least one embodiment according to the invention for the ECG signal in Fig. 5, in accordance the procedure presented for the ECG signal in Fig. 1, Fig. 2, Fig. 3 and Fig. 4.

**Table 4**

| **N** | **S_{N,i} (reference signs)** | **CD_{N,i} (0 or not 0)** | **CNum_{N}** |
|---|---|---|---|
| 0 | S_{0,1} : 510 and 520 | CD_{0,1} = not 0 | CNum₀ = 6 |
| | S_{0,2}: 520 and 530 | CD_{0,2} = not 0 | |
| | S_{0,3}: 530 and 540 | CD_{0,3} = not 0 | |
| | S_{0,4} : 540 and 550 | CD_{0,4} = not 0 | |
| | S_{0,5}: 550 and 560 | CD_{0,5} = not 0 | |
| | S_{0,6}: 560 and 570 | CD_{0,6} = not 0 | |
| 1 | S_{1,1} : 510 and 530 | CD_{1,1} = not 0 | CNum₁ = 5 |
| | S_{1,2}: 520 and 540 | CD_{1,2} = not 0 | |
| | S_{1,3}: 530 and 550 | CD_{1,3} = not 0 | |
| | S_{1,4}: 540 and 560 | CD_{1,4} = not 0 | |
| | S_{1,5} : 550 and 570 | CD_{1,5} = not 0 | |
| 2 | S_{2,1} : 510 and 540 | CD_{2,1} = not 0 | CNum₂ = 4 |
| | S_{2,2} : 520 and 550 | CD_{2,2} = not 0 | |
| | S_{2,3}: 530 and 560 | CD_{2,3} = not 0 | |
| | S_{2,4} : 540 and 570 | CD_{2,4} = not 0 | |
| 3 | S_{3,1} : 510 and 550 | CD_{3,1} = not 0 | CNum₂ = 3 |
| | S_{3,2} : 520 and 560 | CD_{3,2} = not 0 | |
| | S_{3,3}: 530 and 570 | CD_{3,3} = not 0 | |

| | | | |
|---|---|---|---|
| CNum_{N} = 0 is an indication for the example that a regularity is detected. In the case for Fig. 5, CNum_{N} = 0 has not been computed any N using the invention according to at least one embodiment, meaning that an irregularity such as for the indication of AF has been detected and that the set may be regarded as irregular. According to at least one embodiment of the invention, a quality may be assigned to at least a part of the ECG data, as e.g. tagged as AF, when parameters as shown in Table 4 are computed for a count of consecutive sets. | | | |

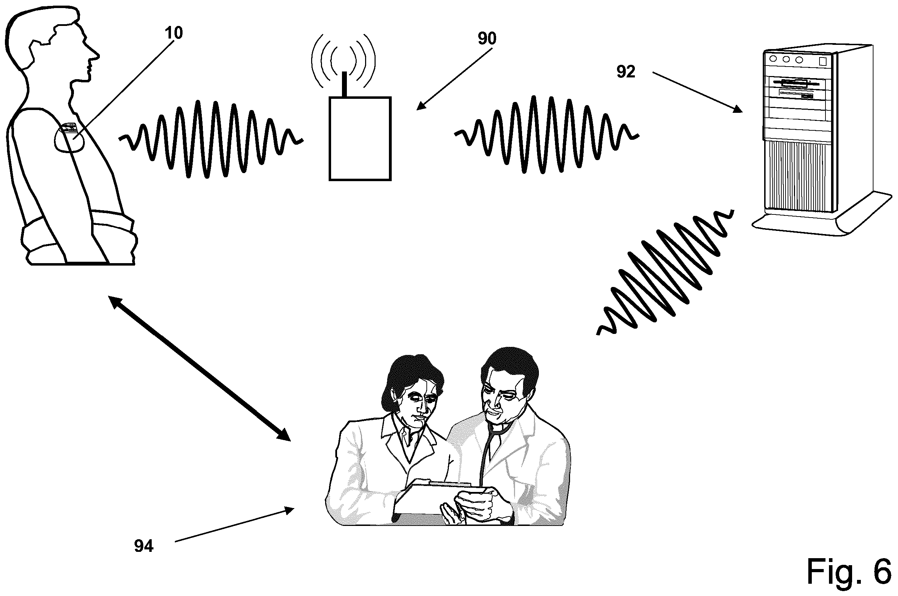

Fig. 6 shows a remote monitoring system and a heart monitor according to one or more embodiments of the invention. As shown in Fig. 6, a remote monitoring system may include one or more of an implantable heart monitor or stimulator 10, an external device 90 and a central data server 92 of a central service center. Such a system, in at least one embodiment, may allow data communication between the implantable heart monitor and stimulator 10 and central server 92 via the external device 90. External device 90, in at least one embodiment, may communicate wirelessly with implantable heart monitor and stimulator 10.

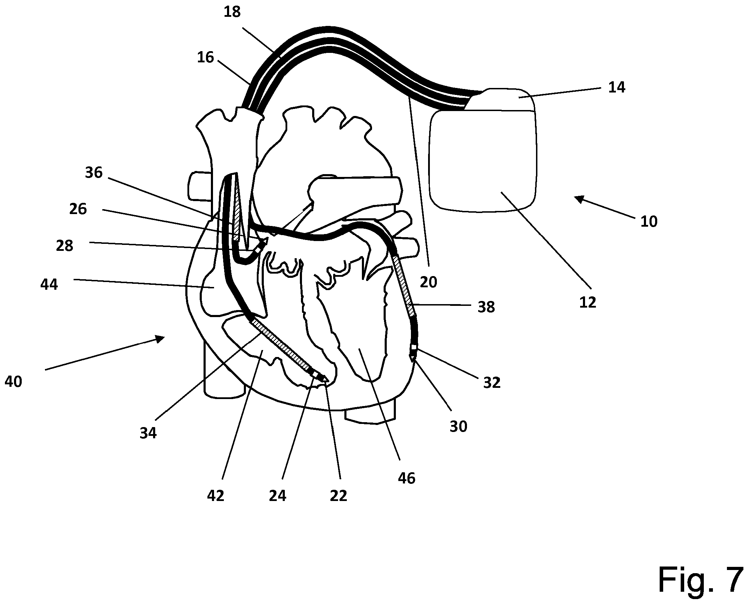

Fig. 7 illustrates a heart stimulator connected to electrode leads that are placed in a heart according to one or more embodiments of the invention. As shown in Fig. 7, the implantable heart monitor and stimulator 10 may include one or more of a housing or case 12 and a header 14.

In at least one embodiment, the implantable heart monitor and stimulator 10 may be connected to three electrode leads, namely a right ventricular electrode lead 16, a right atrial electrode lead 18 and a left ventricular electrode lead 20.

In at least one embodiment of, the heart monitor comprises electrodes without leads.

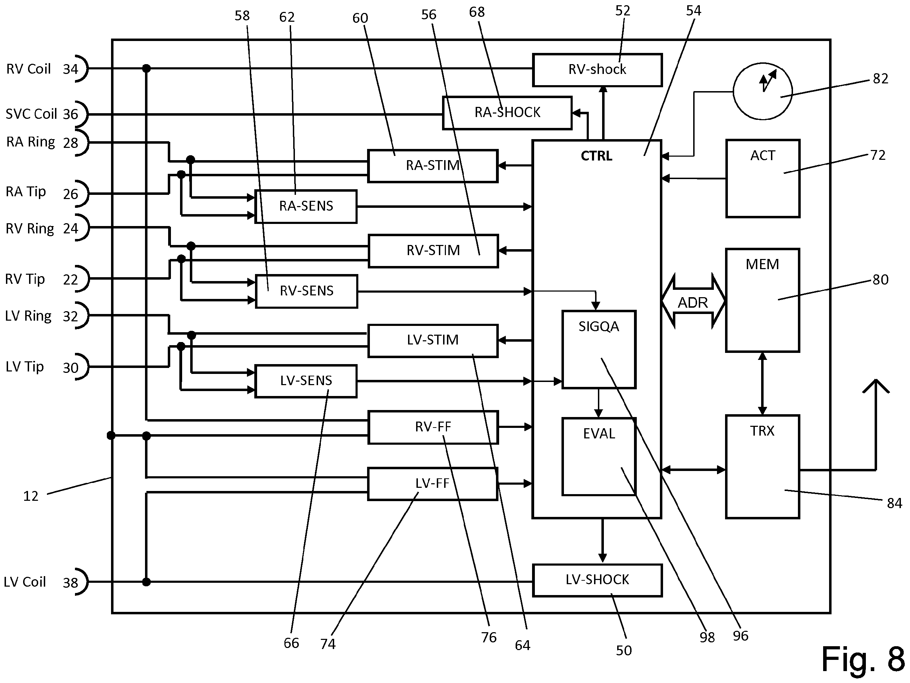

Figs. 7 and 8 illustrate the pacing system that includes the implantable heart monitor and stimulator 10 and the connected leads 16, 18, and 20. In one or more embodiments, the right atrial electrode lead 18 may include one or more of a distal right atrial tip electrode 26 (RA-tip) at the distal end of right atrial electrode lead 18 and a proximal right atrial ring electrode 28 (RA-ring), as well as a superior vena cava coil electrode 36 (SVC-coil) that may have a large surface area.

In one or more embodiments, the right ventricular electrode lead 16 may include one or more of a distal right ventricular tip electrode 22 (RV-tip) at the distal end of right ventricular electrode lead 16 and a proximal right ventricular ring electrode 24 (RV-ring), as well as a right ventricular defibrillation coil electrode 34 (RV-coil) that may have a large surface area.

Similarly, according to at least one embodiment, the left ventricular (LV) lead may include one or more of a distal left ventricular tip electrode 30 (LV-tip) and a proximal left ventricular ring electrode 32 (LV-ring), as well as a defibrillation coil electrode 38 (LV-coil) that has large surface area. The left ventricular electrode lead 20, in at least one embodiment, may pass through the coronary sinus of heart 40.

By way of one or more embodiments, each electrode and shock coil of electrode leads 16 to 20 may be separately connected to an electric circuit enclosed by case 12 of heart stimulator 10 by way of electrical contacts of a plug (not shown) at the proximal end of each electrode lead 16 to 20 and corresponding contacts (not shown) in header 14 of heart stimulator 10.

Fig. 8 depicts a schematic block diagram of some components of the heart stimulator of figure 2 according to one or more embodiments of the invention. As shown in Fig. 8, SVC shock coil 36 may be connected to right atrial shock generator 68 that may be controlled by a control unit 54 of heart stimulator 10.

Similarly, in at least one embodiment of the invention, right ventricular shock coil 34 may be connected to a right ventricular shock generator 52 that may be connected to control unit 54, and left ventricular shock coil 38 may be connected to a left ventricular shock generator 50 that may also be connected to control unit 54.

In one or more embodiments, right atrial tip electrode 26 and right atrial ring electrode 28 may both be connected to a right atrial stimulation pulse generator 60 and a right atrial sensing stage 62, that may internally both be connected to control unit 54.

By way of at least one embodiment, right atrial stimulation pulse generator 60 may generate atrial stimulation pulses of sufficient strength to cause an excitation of atrial myocardium by an electrical pulse delivered via right atrial tip electrode 26 and right atrial ring electrode 28. Preferably, in one or more embodiments, means may adapt the right atrial stimulation pulse strength to the stimulation threshold in the right atrium.

In at least one embodiment, right atrial sensing stage 62 may pick up myocardial potentials indicating an intrinsic atrial excitation that corresponds to a natural atrial contraction. In one or more embodiments, by way of right atrial sensing stage 62, the right atrium 44 of heart 40 in a demand mode may be stimulated, wherein a right atrial stimulation pulse may be inhibited if an intrinsic atrial event (intrinsic atrial excitation) is sensed by right atrial sensing stage 62 prior to expiration of an atrial escape interval.

In a similar manner, in one or more embodiments, right ventricular ring electrode 24 and right ventricular tip electrode 22 may be connected to right ventricular stimulation pulse generator 56 and to a right ventricular sensing stage 58 that in turn may be connected to control unit 54. The right ventricular sensing stage 58, in at least one embodiment, may be further connected to a signal quality analysis unit 96 of the control unit 54, which may determine whether a noise condition (NC) and/or a low signal indication is present for an intrinsic ventricular event sensed by the right ventricular sensing stage 58. By way of one or more embodiments, the signal quality analysis unit 96 may generate a noise condition and/or low signal indication signal and may provide it to an evaluation unit 98, which represents the signal evaluation unit according to at least one of the embodiments of the invention. In at least one embodiment, by way of right ventricular tip electrode 22, right ventricular ring electrode 24, right ventricular stimulation generator 56 and right ventricular sensing stage 58, right ventricular stimulation pulses may be delivered in a demand mode to the right ventricle 42 of heart 40.

In at least one embodiment, in the same way left ventricular tip electrode 30 and left ventricular ring electrode 32 may be connected to the left ventricular stimulation pulse generator 64 and the left ventricular sensing stage 66 that may be connected to signal quality analysis unit 96 of the control unit 54, and that may allow for stimulating a left ventricle 46 of heart 40.

By way of one or more embodiments, the outputs of the ventricular sensing states 58 and 66, i.e., sense signals comprising ventricular events, and of the signal quality analysis unit 96, i.e., noise condition and/or low signal indication signals, may be provided to the evaluation unit 98. In at least one embodiment, the evaluation unit 98 may evaluate the signals by detecting useable intervals in the sense signals in dependence of the noise condition and/or low signal indication signals. In embodiments of the invention, useable intervals may be intervals, which may be defined by two consecutive events that do not have a noise condition and/or a low signal indication. The evaluation unit 98, in one or more embodiments, may execute various monitoring and stimulation algorithms in parallel to monitor specific heart functional and rhythm disorders, e.g., bradycardia, asystole, high ventricular rate, or other heart functional or rhythm disorders and to treat the disorder, e.g., by stimulating the left ventricle 46 and/or the right ventricle 42 of heart 40. The evaluation unit 98, in at least one embodiment, may determine an average interval duration and an average rate of events from the useable intervals and may use these parameters to detect bradycardia, asystole, and/or high ventricular rate. If a functional disorder has been detected, in one or more embodiments, the evaluation unit 98 may execute an alternative monitoring and stimulation algorithm for the specific functional disorder which has been detected. In at least one embodiment, the alternative monitoring and stimulation algorithm may attempt to detect, whether the functional disorder was terminated. A termination may occur, in one or more embodiments, caused by stimulating the left ventricle 46 or right ventricle 42 of heart 40. The detection of termination and a stimulation adjusted to a detected functional disorder, in at least one embodiment, may also be integrated in one monitoring and stimulation algorithm.

According to at least one embodiment, triggering and inhibition of delivery of stimulation pulses to the right atrium, the right ventricle or the left ventricle may be controlled by control unit 54. The timing that schedules delivery of stimulation pulses if needed, in at least one embodiment, may be controlled by a number of intervals that at least partly may depend on a hemodynamic demand of a patient that may be sensed using an activity sensor 72 that may be connected to control unit 54. Activity sensor 72, in at least one embodiment, may allow for rate adaptive pacing wherein a pacing rate (the rate of consecutive ventricular stimulation pulses for a duration of consecutive atrial stimulation pulses) may depend on a physiological demand of a patient that may be sensed by a way of activity sensor 72.

In one or more embodiments, a clock 82 may allow recording of events and signals in association with time stamps that may enable a synchronous evaluation of signals at a later point of time.

By way of at least one embodiment, for the purpose of composition of a far-field right ventricular electrogram (RV EGM) and a far-field left-ventricular electrogram (LV EGM) a far-field right ventricular electrogram recording unit 76 and a far-field left ventricular recording unit 74, respectively, may be provided. The far-field right ventricular electrogram recording unit 76, in at least one embodiment, may be connected to a case electrode that may be formed by at least an electrically conducting part of case 12 of the heart stimulator 10 and to the RV coil electrode 34. The far-field left ventricular recording unit 74, in one or more embodiments, may also be connected to the case electrode formed by a case 12 of heart stimulator 10 and to the left ventricular coil electrode 38.

In at least one embodiment, the near-field electrogram in the right ventricle 42 may be measured between the RV-tip electrode 22 and RV-ring electrode 24. Preferably, in one or more embodiments, the far-field electrogram in the right ventricle 42 may be measured between the RV-coil electrode 34 and the device housing 12. Alternatively, in at least one embodiment, the far-field electrogram in the right ventricle 42 may be measured between the RV-ring electrode 24 and the device housing 12.

Likewise, in one or more embodiments, the near-field electrogram in the left ventricle 46 may be measured between the LV-tip electrode 30 and LV-ring electrode 32. Preferably, in at least one embodiment, the far-field electrogram in left ventricle may be measured between the LV-coil electrode 38 and the device housing 12. Alternatively, in embodiments of the invention, the far-field electrogram in the left ventricle 46 may be measured between the LV-ring electrode 32 and the device housing 12.

In at least one embodiment, preferably, the far-field electrogram in the right ventricle 42 and the left ventricle 46 may be minimally filtered and have wide bandwidth, e.g., with lower corner frequency 4 Hz and high corner frequency 128 Hz, whereas the near-field electrograms in the right ventricle 42 and the left ventricle 46 may be filtered with narrower bandwidth, e.g., with lower corner frequency 18 Hz and high corner frequency 40 Hz. Accordingly, in one or more embodiments, right and left far-field ventricular recording units 76 and 74 may each include a band pass filter with lower corner frequency 4 Hz and high corner frequency 128 Hz. Right ventricular sensing stage 58 and left ventricular sensing stage 66 for picking up near-field electrograms in the right ventricle 42 and the left ventricle 46, according to at least one embodiment, may each include band-pass filters with narrower bandwidth, e.g., with lower corner frequency 18 Hz and high corner frequency 40 Hz.

Both the far-field electrograms and the near-field electrograms, in one or more embodiments, may be used to detect events in the signals and to determine intervals and/or a corresponding a rate of events. In at least one embodiment, the signal quality analysis unit 96 may determine whether a noise condition (NC) and/or a low signal indication may be present for an intrinsic event sensed by the sensing stages 58, 66 and/or the far-field ventricular electrogram recording units 74, 76. The corresponding noise condition (NC) and/or low signal indication signal, in one or more embodiments, may be provided to the evaluation unit 98. In at least one embodiment, the evaluation unit 98 may evaluate the outputs of the sensing stages 58, 66 and the far-field ventricular electrogram recording units 74, 76 in dependence of the noise condition (NC) and/or low signal indication signal, i.e., determining an average interval duration and an average rate of events from the useable intervals and using these parameters to detect bradycardia, asystole, and/or high ventricular rate.

According to at least one embodiment of the invention, the heart monitor 10 may be an implantable device, used as a loop recorder, that detects QRS complexes using the subcutaneous electrodes 22, 24, 30, 32 as shown in Fig. 7 and Fig. 8. In one or more embodiments, the heart monitor 10 may combine different electrode measurements to create a combined signal and then may perform QRS detection on the combined signal. In at least one embodiment, the detected QRS events may be classified as ventricular sense events (VS) 102 or as invalid sensed events (VN) 104. A VS 102, in at least one embodiment, may be considered a VN 104 if it has an associated noise condition (NC) or low signal indication.

The invention is defined in the following claims. Other embodiments, examples, items etc. are not a part of the invention.

## Claims

1. A computer-implemented method for analyzing ECG data, comprising
- detecting points in the ECG data which represent ventricular activity (101),
- measuring time intervals between each two consecutive points in the ECG data which represent ventricular activity (101),
- evaluating the time intervals within a set of time intervals by
- computing at least one comparative dimension for at least one time interval subset,
**characterized in that**
- the set of time intervals comprises at least three time intervals,
- the time interval subset comprises at least two time intervals,
- the comparative dimension represents a difference of the interval length between the time intervals of the time interval subset, and wherein
- a fixed number N of time intervals lies between each two time intervals of the time interval subset and wherein N ≥ 1.

2. The method according to claim 1, wherein within the set of time intervals, an additional comparative dimension for at least one time interval subset is computed for which N = 0.

3. The method according to one of the preceding claims, wherein
- within the set of time intervals, all computed comparative dimensions with equal N are considered as a group, and
- for each group, a number is calculated for those comparative dimensions which fulfil an instability criterion.

4. The method according to claim 3, wherein
- each calculated number is compared with at least one threshold respectively and
- the set of time intervals is regarded as irregular, when
- at least one calculated number exceeds at least one threshold or when
- all calculated numbers exceed at least one threshold respectively.

5. The method according to claim 4, wherein
- a quality is assigned to at least a part of the ECG data, when
- a count of consecutive sets of time intervals which are regarded as irregular exceeds a value A and/or when
- within a totality X of sets of time intervals, the count of consecutive sets of time intervals which are regarded as irregular exceeds the value A.

6. The method according to claim 5, wherein
- the assignment of a quality to at least a part of the ECG data is terminated, when
- a count of consecutive sets of time intervals which are regarded as not irregular exceeds a value B and/or when
- within a totality Y of sets of time intervals, the count of consecutive sets of time intervals which are regarded as not irregular exceeds the value B.

7. The method according to one of the claims 3 - 6, wherein the instability criterion is fulfilled, when the comparative dimension exceeds a limit.

8. The method according to one of the preceding claims, wherein the comparative dimension is computed by generation of at least a difference, a sum, a ratio, a product, a mean value, a deviation, or a variance.

9. The method according to one of the preceding claims, wherein the set of time intervals comprises at least three time intervals.

10. Device for monitoring the heart activity of a living being (10), comprising
- at least two electrodes (16, 18, 20, 26, 28, 36, 22, 24, 34, 30, 32, 38) for recording a signal which represents the heart activity,
- a power supply,
- a memory unit for storing the signal (80),
- a signal evaluation unit (98), which is configured for
- detecting ventricular activity in the signal (101),
- measuring time intervals between each two consecutive ventricular activities (101),
- evaluating the time intervals within a set of time intervals by
- computing at least one comparative dimension for at least one time interval subset,
**characterized in that**
- the set of time intervals comprises at least three time intervals,
- the time interval subset comprises at least two time intervals,
- the comparative dimension represents a difference of the interval length between the time intervals of the time interval subset, and wherein
- a fixed number N of time intervals lies between each two time intervals of the time interval subset and wherein N ≥ 1.

11. The device according to claim 10, wherein the signal evaluation (98) unit is further configured to compute an additional comparative dimension for which N =0 for at least one time interval subset within the set of time intervals.

12. The device according to one of the claims 10 or 11, wherein the signal evaluation unit (98) is further configured to
- consider all computed comparative dimensions with equal N within the set of time intervals as a group, and
- calculate a number for each group for those comparative dimensions which fulfil at least one instability criterion.

13. The device according to claim 12, wherein the signal evaluation unit (98) is further configured to
- regard the set of time intervals as irregular, when
- at least one calculated number exceeds at least one threshold or when
- all calculated numbers exceed at least one threshold.

14. The device according to claim 13, wherein the signal evaluation unit (98) is further configured to
- recognize a pathological state of the heart activity, when
- a count of consecutive sets of time intervals which are regarded as irregular exceeds a value A and/or when
- within a totality X of sets of time intervals, the count of consecutive sets of time intervals which are regarded as irregular exceeds the value A.

15. The device according to claim 14, wherein the signal evaluation unit (98) is further configured to
- terminate the recognition of a pathological state of the heart activity, when
- a count of consecutive sets of time intervals which are regarded as not irregular exceeds a value B and/or when
- within a totality Y of sets of time intervals, the count of consecutive sets of time intervals which are regarded as not irregular exceeds the value B.

## Patentansprüche

1. Computer-implementiertes Verfahren zur Analyse von EKG-Daten, umfassend
- Detektion von Punkten in den EKG-Daten, die ventrikuläre Aktivität (101) darstellen,
- Messung von Zeitintervallen zwischen je zwei aufeinanderfolgenden Punkten in den EKG-Daten, die ventrikuläre Aktivität (101) darstellen,
- Auswertung der Zeitintervalle innerhalb eines Satzes von Zeitintervallen durch
- Berechnung von mindestens einer vergleichenden Dimension für mindestens einen Zeitintervall-Teilsatz,
**dadurch gekennzeichnet, dass**
- der Satz von Zeitintervallen mindestens drei Zeitintervalle umfasst,
- der Zeitintervall-Teilsatz mindestens zwei Zeitintervalle umfasst,
- die vergleichende Dimension eine Differenz der Intervalllänge zwischen den Zeitintervallen des Zeitintervall-Teilsatzes darstellt, und wobei
- eine feste Zahl N von Zeitintervallen zwischen je zwei Zeitintervallen des Zeitintervall-Teilsatzes liegt und wobei N ≥ 1.

2. Verfahren nach Anspruch 1, wobei innerhalb des Satzes von Zeitintervallen eine zusätzliche vergleichende Dimension für mindestens einen Zeitintervall-Teilsatz für N = 0 berechnet wird.

3. Verfahren nach einem der vorstehenden Ansprüche, wobei
- innerhalb des Satzes von Zeitintervallen alle berechneten vergleichenden Dimensionen mit gleichem N als Gruppe betrachtet werden, und
- für jede Gruppe eine Zahl für solche vergleichenden Dimensionen berechnet wird, die ein Instabilitätskriterium erfüllen.

4. Verfahren nach Anspruch 3, wobei
- jede berechnete Zahl mit je mindestens einem Schwellenwert abgeglichen wird, und
- der Satz von Zeitintervallen als unregelmäßig betrachtet wird, wenn
- mindestens eine berechnete Zahl mindestens einen Schwellenwert übersteigt, oder wenn
- alle berechneten Zahlen je mindestens einen Schwellenwert übersteigen.

5. Verfahren nach Anspruch 4, wobei
- mindestens einem Teil der EKG-Daten eine Qualität zugewiesen wird, wenn
- eine Zählung aufeinanderfolgender Sätze von Zeitintervallen, die als unregelmäßig betrachtet werden, einen Wert A übersteigt, und/oder wenn
- in einer Gesamtheit X an Sätzen von Zeitintervallen die Zählung aufeinanderfolgender Sätze von Zeitintervallen, die als unregelmäßig betrachtet werden, den Wert A übersteigt.

6. Verfahren nach Anspruch 5, wobei
- die Zuweisung einer Qualität an mindestens einen Teil der EKG-Daten beendet ist, wenn
- eine Zählung aufeinanderfolgender Sätze von Zeitintervallen, die als nicht unregelmäßig betrachtet werden, einen Wert B übersteigt und/oder wenn
- in einer Gesamtheit Y an Sätzen von Zeitintervallen die Zählung aufeinanderfolgender Sätze von Zeitintervallen, die nicht als unregelmäßig betrachtet werden, den Wert B übersteigt.

7. Verfahren nach einem der Ansprüche 3 bis 6, wobei das Instabilitätskriterium erfüllt ist, wenn die vergleichende Dimension einen Schwellenwert übersteigt.

8. Verfahren nach einem der vorstehenden Ansprüche, wobei die vergleichende Dimension durch Generierung von mindestens einer Differenz, einer Summe, einem Verhältnis, einem Produkt, einem Durchschnittswert, einer Abweichung oder einer Varianz berechnet wird.

9. Verfahren nach einem der vorstehenden Ansprüche, wobei der Satz von Zeitintervallen mindestens drei Zeitintervalle umfasst.

10. Gerät zur Überwachung der Herztätigkeit eines Lebewesens (10), umfassend:
- mindestens zwei Elektroden (16, 18, 20, 26, 28, 36, 22, 24, 34, 30, 32, 38) zur Aufzeichnung eines Signals, das die Herztätigkeit darstellt,
- eine Stromversorgung,
- eine Speichereinheit zum Speichern des Signals (80),
- eine Signalauswertungseinheit (98), die dafür ausgelegt ist,
- eine ventrikuläre Aktivität im Signal zu detektieren (101),
- Zeitintervalle zwischen je zwei aufeinanderfolgenden ventrikulären Aktivitäten (101) zu messen,
- die Zeitintervalle in einem Satz von Zeitintervallen auszuwerten, durch
- Berechnung von mindestens einer vergleichenden Dimension für mindestens einen Zeitintervall-Teilsatz,
**dadurch gekennzeichnet, dass**
- der Satz von Zeitintervallen mindestens drei Zeitintervalle umfasst,
- der Zeitintervall-Teilsatz mindestens zwei Zeitintervalle umfasst,
- die vergleichende Dimension eine Differenz der Intervalllänge zwischen den Zeitintervallen des Zeitintervall-Teilsatzes darstellt, und wobei
- eine feste Zahl N von Zeitintervallen zwischen je zwei Zeitintervallen des Zeitintervall-Teilsatzes liegt und wobei N ≥ 1.

11. Verfahren nach Anspruch 10, wobei die Signalauswertungseinheit (98) ferner dafür ausgelegt ist, eine zusätzliche vergleichende Dimension, für mindestens einen Zeitintervall-Teilsatz innerhalb des Satzes von Zeitintervallen, für N = 0 zu berechnen.

12. Gerät nach einem der Ansprüche 10 oder 11, wobei die Signalauswertungseinheit (98) ferner dafür ausgelegt ist
- alle berechneten vergleichenden Dimensionen mit gleichem N innerhalb des Satzes von Zeitintervallen als eine Gruppe zu betrachten, und
- für jede Gruppe eine Zahl für solche vergleichenden Dimensionen zu berechnen, die mindestens ein Instabilitätskriterium erfüllen.

13. Gerät nach Anspruch 12, wobei die Signalauswertungseinheit (98) ferner dafür ausgelegt ist
- den Satz von Zeitintervallen als unregelmäßig zu betrachten, wenn
- mindestens eine berechnete Zahl mindestens einen Schwellenwert übersteigt oder wenn
- alle berechnete Zahlen mindestens einen Schwellenwert übersteigen.

14. Gerät nach Anspruch 13, wobei die Signalauswertungseinheit (98) ferner dafür ausgelegt ist
- einen pathologischen Zustand der Herztätigkeit zu erkennen, wenn
- eine Zählung aufeinanderfolgender Sätze von Zeitintervallen, die als unregelmäßig betrachtet werden, einen Wert A übersteigt, und/oder wenn
- in einer Gesamtheit X an Sätzen von Zeitintervallen die Zählung aufeinanderfolgender Sätze von Zeitintervallen, die als unregelmäßig betrachtet werden, den Wert A übersteigt.

15. Gerät nach Anspruch 14, wobei die Signalauswertungseinheit (98) ferner dafür ausgelegt ist
- die Erkennung eines pathologischen Zustands der Herztätigkeit zu beenden, wenn
- eine Zählung aufeinanderfolgender Sätze von Zeitintervallen, die als nicht unregelmäßig betrachtet werden, einen Wert B übersteigt, und/oder wenn
- in einer Gesamtheit Y an Sätzen von Zeitintervallen die Zählung aufeinanderfolgender Sätze von Zeitintervallen, die nicht als unregelmäßig betrachtet werden, den Wert B übersteigt.

## Revendications

1. Procédé mis en œuvre par ordinateur pour analyser des données d'ECG, comprenant
- la détection de points dans les données d'ECG qui représentent une activité ventriculaire (101),
- la mesure d'intervalles de temps chaque fois entre deux points consécutifs dans les données d'ECG qui représentent l'activité ventriculaire (101),
- l'évaluation des intervalles de temps dans un ensemble d'intervalles de temps en
- calculant au moins une dimension comparative pour au moins un sous-ensemble d'intervalle de temps,
**caractérisé en ce que**
- l'ensemble d'intervalles de temps comprend au moins trois intervalles de temps,
- le sous-ensemble d'intervalle de temps comprend au moins deux intervalles de temps,
- la dimension comparative représente une différence de la longueur d'intervalle entre les intervalles de temps du sous-ensemble d'intervalle de temps, et dans lequel
- un nombre fixe N d'intervalles de temps se situe chaque fois entre deux intervalles de temps du sous-ensemble d'intervalle de temps et dans lequel N ≥ 1.

2. Procédé selon la revendication 1, dans lequel, dans l'ensemble d'intervalles de temps, une dimension comparative supplémentaire pour au moins un sous-ensemble d'intervalle de temps est calculée pour lequel N = 0.

3. Procédé selon l'une des revendications précédentes, dans lequel
- dans l'ensemble d'intervalles de temps, toutes les dimensions comparatives calculées avec un N égal sont considérées comme un groupe, et
- pour chaque groupe, un nombre est calculé pour les dimensions comparatives en question qui satisfont à un critère d'instabilité.

4. Procédé selon la revendication 3, dans lequel
- chaque nombre calculé est comparé respectivement avec au moins un seuil et
- l'ensemble d'intervalles de temps est considéré comme irrégulier lorsque
- au moins un nombre calculé dépasse au moins un seuil ou lorsque
- tous les nombres calculés dépassent respectivement au moins un seuil.

5. Procédé selon la revendication 4, dans lequel
- une qualité est associée à au moins une partie des données d'ECG lorsque
- un comptage d'ensembles consécutifs d'intervalles de temps qui sont considérés comme irréguliers dépasse une valeur A et/ou lorsque
- dans une totalité Y d'ensembles d'intervalles de temps, le comptage d'ensembles consécutifs d'intervalles de temps qui sont considérés comme non irréguliers dépasse la valeur A.

6. Procédé selon la revendication 5, dans lequel
- l'association d'une qualité à au moins une partie des données d'ECG est réalisée lorsque
- un comptage d'ensembles consécutifs d'intervalles de temps qui sont considérés comme non irréguliers dépasse une valeur B et/ou lorsque
- dans une totalité Y d'ensembles d'intervalles de temps, le comptage d'ensembles consécutifs d'intervalles de temps qui sont considérés comme non irréguliers dépasse la valeur B.

7. Procédé selon l'une des revendications 3 à 6, dans lequel le critère d'instabilité est satisfait lorsque la dimension comparative dépasse une limite.

8. Procédé selon l'une des revendications précédentes, dans lequel la dimension comparative est calculée par la génération d'au moins une différence, d'une somme, d'un ratio, d'un produit, d'une valeur moyenne, d'une déviation ou d'une variance.

9. Procédé selon l'une des revendications précédentes, dans lequel l'ensemble d'intervalles de temps comprend au moins trois intervalles de temps.

10. Dispositif de suivi de l'activité cardiaque d'un être vivant (10) comprenant
- au moins deux électrodes (16, 18, 20, 26, 28, 36, 22, 24, 34, 30, 32, 38) pour enregistrer un signal que représente une activité cardiaque,
- une alimentation en énergie,
- une unité mémoire pour l'enregistrement du signal (80),
- une unité d'évaluation de signal (98) qui est conçue pour
- détecter une activité ventriculaire dans le signal (101),
- mesurer des intervalles de temps chaque fois entre deux activités ventriculaires (101) consécutives,
- évaluer les intervalles de temps dans un ensemble d'intervalles de temps en
- calculant au moins une dimension comparative pour au moins un sous-ensemble d'intervalle de temps,
**caractérisé en ce que**
- l'ensemble d'intervalles de temps comprend au moins trois intervalles de temps,
- le sous-ensemble d'intervalles de temps comprend au moins deux intervalles de temps,
- la dimension comparative représente une différence de la longueur d'intervalle entre les intervalles de temps du sous-ensemble d'intervalle de temps, et dans lequel
- un nombre fixe N d'intervalles de temps se situe chaque fois entre deux intervalles de temps du sous-ensemble d'intervalle de temps et dans lequel N ≥ 1.

11. Dispositif selon la revendication 10, dans lequel l'unité d'évaluation de signal (98) est en outre conçue pour calculer une dimension comparative supplémentaire pour laquelle N = 0 pendant au moins un sous-ensemble d'intervalle de temps dans l'ensemble des intervalles de temps.

12. Dispositif selon l'une des revendications 10 ou 11, dans lequel l'unité d'évaluation de signal (98) est en outre conçue pour
- prendre en considération toutes les dimensions comparatives calculées avec un N égal dans l'ensemble des intervalles de temps sous forme d'un groupe, et
- calculer un nombre pour chaque groupe pour les dimensions comparatives en question qui satisfont au moins un critère d'instabilité.

13. Dispositif selon la revendication 12, dans lequel l'unité d'évaluation de signal (98) est en outre conçue pour
- considérer l'ensemble d'intervalles de temps comme irrégulier lorsque
- au moins un nombre calculé dépasse au moins un seuil ou lorsque
- tous les nombres calculés dépassent au moins un seuil.

14. Dispositif selon la revendication 13, dans lequel l'unité d'évaluation de signal (98) est en outre conçue pour
- reconnaître un état pathologique de l'activité cardiaque, lorsque
- un comptage d'ensembles consécutifs d'intervalles de temps qui sont considérés comme irréguliers dépasse une valeur A et/ou lorsque
- dans une totalité X d'ensembles d'intervalles de temps, le comptage d'ensembles consécutifs d'intervalles de temps qui sont considérés comme irréguliers dépasse la valeur A.

15. Dispositif selon la revendication 14, dans lequel l'unité d'évaluation de signal (98) est en outre conçue pour
- réaliser la reconnaissance d'un état pathologique de l'activité cardiaque lorsque
- un comptage d'ensembles consécutifs d'intervalles de temps qui sont considérés comme non irréguliers dépasse une valeur B et/ou lorsque
- dans une totalité Y d'ensembles d'intervalles de temps, le comptage d'ensembles consécutifs d'intervalles de temps qui sont considérés comme non irréguliers dépasse la valeur B.
